# EUROPÄISCHE PATENTSCHRIFT

(11) **EP 3 609 548 B1**
(45) Veröffentlichungstag und Bekanntmachung des Hinweises auf die Patenterteilung: **16.03.2022**
(21) Anmeldenummer: 18719453.5
(22) Anmeldetag: 03.04.2018
(51) Int. Cl.: A61L 27/34, A61L 27/36, A61L 27/38, A61L 27/54, A61L 2/04, A61L 2/00

(54) **NATIVES KNOCHENERSATZMATERIAL ZUR FÖRDERUNG DER OSTEOGENESE, VERFAHREN ZU DESSEN HERSTELLUNG UND VERWENDUNGEN**
NATIVE BONE SUBSTITUTE MATERIAL FOR PROMOTING OSTEOGENESIS, METHOD FOR THE PRODUCTION THEREOF, AND USES
SUBSTITUT OSSEUX NATIF DESTINÉ À FAVORISER L'OSTÉOGENÈSE, SON PROCÉDÉ DE FABRICATION ET SON UTILISATION

(30) Priorität: 13.04.2017 DE 102017206453
(43) Veröffentlichungstag der Anmeldung: 19.02.2020
(73) Patentinhaber: Bioimplon GmbH, 35398 Giessen (DE)
(72) Erfinder: WATAD, Sami, 35444 Biebertal (DE)
(74) Vertreter: advotec.
(86) Internationale Anmeldenummer: PCT/EP2018/058455
(87) Internationale Veröffentlichungsnummer: WO 2018/188989

(56) Entgegenhaltungen:
- WO-A2-2008/125858
- WO-A2-2011/018057

## Beschreibung

Die Erfindung betrifft ein natives Knochenersatzmaterial zur Förderung der Osteogenese sowie ein Verfahren zu dessen Herstellung und Verwendungen nach dem Oberbegriff der unabhängigen Ansprüche.

Die Behandlung diverser skelettaler Defekte, insbesondere im Bereich der Mund-, Kiefer- und/oder Gesichtschirurgie, der zahnärztlichen Chirurgie, der Traumatologie, der Orthopädie, der Implantologie und/oder der Parodontologie, besitzt eine lange und erfolgreiche Tradition. Traditionell wird dafür autogener, isogener oder allogener Knochen, teils in verarbeitetem Zustand, übertragenen, was jedoch mit einigen Nachteilen verbunden ist. So kommt es für einen Patienten, beispielsweise, nicht nur zu einer drastischen Verlängerung des chirurgischen Eingriffs, sondern auch zu einer erhöhten Morbidität und einem signifikant erhöhten Risiko der Übertragung von Infektionskrankheiten. Zudem ist autologer, isogener, sowie auch allogener Knochen nicht in unbegrenzten Volumina verfügbar.

Es ist daher nicht verwunderlich, dass der Einsatz und die Verwendung von Knochenersatzmaterialien in der Behandlung skelettaler Defekte zunehmend an Bedeutung gewinnen. Knochenersatzmaterialien stellen eine attraktive Alternative zum autogenen, isogenen und allogenen Knochen dar, insbesondere aufgrund der Vermeidung der Entnahmemorbidität und der möglichen Bereitstellung in unbegrenzten Volumina. Die Anforderungen an ein ideales Knochenersatzmaterial sind verständlicherweise hoch. Diese sind beispielsweise, jedoch keinesfalls ausschließlich, die Gewährleistung und Sicherstellung der Biokompatibilität, die Osteoinduktion, die Osteokonduktion, die anforderungsgemäße Porosität, die Belastungsstabilität, die Resorbierbarkeit, die sterile Formbarkeit und/oder die Gewährleistung und Sicherstellung der stabilen und langfristigen Integration von Implantaten.

Aus dem aktuellen Stand der Technik sind eine Vielzahl verschiedener Knochenersatzmaterialien bekannt, wie beispielweise xenogene Knochenersatzmaterialien, alloplastische Knochenersatzmaterialien und Kompositematerialien. Unter diesen ist poröses Hydroxylapatit das bevorzugteste, da es einen natürlichen Bestandteil des anorganischen Knochengewebes darstellt, sowie eine sehr gute Gewebeverträglichkeit und knochenbildende Eigenschaften aufweist. Zudem erfüllt Hydroxylapatit alle Anforderungen, die bezüglich Histokompatibilität an ein Knochenersatzmaterial gestellt werden müssen. Das heißt, dass Hydroxylapatit, wie in mehreren Untersuchungen nachgewiesen wurde, weder immunogen, noch toxisch und/oder kanzerogen wirkt.

WO2011/018057 A offenbart ein medizinisches Produkt zur Verbesserung der Knochenbildung auf Basis eines nativen Kompositmaterials mit einer Hydroxyapatitstruktur mit Kollagen, wobei die Telopeptide des Kollagen mindestens teilweise durch enzymatische Atelopeptidation entfernt worden sind. Hierzu wird ein proteolytisches Enzym ohne kollagenolytische Aktivität, bevorzugt Pepsin, eingesetzt. Das Produkt kann durch Gammabestrahlung sterilisiert werden.

Da prinzipiell bei allen biologisch gewonnenen Knochenersatzmaterialien, mit Ausnahme der autogenen Transplantate, die Gefahr der Krankheitsübertragung und/oder der Immunogenität besteht, welche sich durch Abstoßung und/oder Allergie bei einem Patienten äußern kann, ist es bei diesen Knochenersatzmaterialien besonders wichtig, möglichst alle organischen Bestandteile zu beseitigen und damit mögliche immunologische Abwehrreaktionen vorzubeugen bzw. zu reduzieren. So ist es beispielsweise bekannt, die organischen Bestandteile der natürlichen Knochenersatzmaterialien durch chemische, biologische, physiochemische und/oder physikalische Verfahren zu beseitigen. Die aus dem Stand der Technik bekannten Knochenersatzmaterialien weisen alle den Nachteil auf, dass vor allem deren Biokompatibilität nicht vollständig gewährleistet werden kann, so dass es bei einer Vielzahl der Anwendungsfälle nötig ist, einem Patienten gleichzeitig zu der Behandlung mit dem Knochenersatzmaterial antibiotische und/oder immunsupressive Mittel zu verabreichen, um eine allergische Reaktion und/oder Abstoßungsreaktion zu verhindern bzw. zu unterdrücken. Dies ist verständlicherweise mit einer unnötigen Belastung des Patienten, sowie unnötigen Kosten verbunden.

Es besteht daher ein großer Bedarf an einem nativen Knochenersatzmaterial, welches zuverlässig, schnell und langzeitstabil für die prophylaktische und/oder therapeutische Behandlung diverser skelettaler Defekte verwendet werden kann, um eine Verbesserung des Zustandes und eine mechanische Stabilisierung der Knochen- und/oder Weichgewebesituation zu erreichen, so dass der entsprechende Defekt bevorzugt mit regeneriertem, vitalem Knochen ausheilt. Zudem sollte das native Knochenersatzmaterial in höchstem Maße biokompatibel sein und die eingangs genannten Anforderungen erfüllen. Überdies sollte das native Knochenersatzmaterial kostengünstig herstellbar, langzeitspeicherbar und individuell an die entsprechende prophylaktische und/oder therapeutische Behandlung anpassbar sein. Die Erfindung hat sich daher die Aufgabe gestellt, ein natives Knochenersatzmaterial zur Förderung der Osteogenese bereitzustellen, um die oben genannten Schwierigkeiten zu überwinden und um vor allem ein höchstes Maß an Biokompatibilität bei gleichzeitig bestmöglicher und schneller Integration in den Knochen und geringen Herstellungskosten zu gewährleisten.

Diese Aufgabe wird auf überraschend einfache, aber wirkungsvolle Weise durch ein natives Knochenersatzmaterial zur Förderung der Osteogenese, sowie ein Verfahren zu dessen Herstellung und Verwendung nach der Lehre der unabhängigen Hauptansprüche gelöst.

Erfindungsgemäß ist ein Verfahren zur Herstellung eines nativen Knochenersatzmaterials vorgeschlagen, welches die Schritte umfasst:
(a) Zerkleinern und Reinigen eines nativen Knochenverbundmaterials, welches eine Hydroxylapatit-Struktur aufweist und Kollagen enthält; und
(b) Auftragen eines proteolytischen Enzyms auf das Knochenverbundmaterial aus Schritt (a), wobei das Kollagen atelopeptidiert wird; und
(c) Sterilisieren des Knochenverbundmaterials aus Schritt (b), wobei
   die Sterilisation bei einer Temperatur von 250 °C bis 500 °C
   durchgeführt wird; und
(d) Erhalten des Knochenersatzmaterials.

Das erfindungsgemäße Verfahren beruht auf dem Grundgedanken, dass die organischen Komponenten das nativen Knochenverbundmaterials aufgrund der Kombination der Atelopeptidierung des in dem Knochenverbundmaterial enthalten Kollagens, bevorzugt des Typ I Kollagens, und der anschließenden thermischen Sterilisation desselben vollständig entfernt werden. Dabei ist es als wesentlich erkannt worden, dass die chemischen, biologischen, biochemischen, mechanischen, biomechanischen und/oder physikalischen Eigenschaften des Knochenverbundmaterials trotz dessen Atelopeptidierung und Sterilisierung unverändert bleiben. Auf diese Weise wird es gewährleistet, dass ein sicheres, peptidfreies und gleichzeitig kollagenhaltiges natives Knochenersatzmaterial erhältlich ist, welches bioaktiv ist und die eingangs genannten bevorzugten Anforderungen hinsichtlich der Gewährleistung und Sicherstellung der Biokompatibilität, der Osteokonduktion, der anforderungsgemäßen Porosität, der Belastungsstabilität, der Resorbierbarkeit, der sterilen Formbarkeit und/oder der Gewährleistung und Sicherstellung der stabilen und langfristigen Integration von Implantaten aufweist.

Der Begriff "natives Knochenverbundmaterial" betrifft eine von tierischem, pflanzlichem und/oder menschlichem Gewebe stammende, biologisch-organische Verbindung, welche eine Hydroxylapatit-Struktur aufweist und Kollagen enthält. Bevorzugt ist das Kollagen Typ I Kollagen.

Der Begriff "natives Knochenersatzmaterial" betrifft eine künstlich erzeugte, von einem tierischen, pflanzlichen und/oder menschlichen Gewebe stammende, bioaktive, biologisch-anorganische Verbindung. Das native Knochenersatzmaterial weist zudem die eingangs genannten bevorzugten Anforderungen auf. Der Begriff "bioaktiv" bedeutet im Rahmen der vorliegenden Erfindung, dass eine knöcherne Integration und damit verbunden eine feste Verbindung zwischen dem vitalen Knochen und dem Knochenersatzmaterial erreicht wird.

Der Betriff "proteolytisches Enzym" ist einem Fachmann gut bekannt und betrifft die enzymatische Hydrolyse von Proteinen durch Peptidasen. Im vorliegenden Fall ist es dabei erkannt worden, dass das proteolytische Enzym bevorzugt keine kollagenolytische Aktivität aufweist. Bevorzugt ist das proteolytische Enzym Pepsin, Trypsin, Pronase und/oder Proctase.

Der Begriff "Atelopeptidierung" betrifft die enzymatische, physiochemische Entfernung der immunogenen terminalen Peptidkette innerhalb des Kollagenmoleküls, bevorzugt des sogenannten Telopeptids. Bevorzugt wird diese terminale Peptidkette derart entfernt, so dass lediglich die natürliche und native Kollagenstruktur in dem Knochenverbundmaterial bestehen bleibt. Mittels der Atelopeptidierung ist es somit möglich, ein modifiziertes Kollagen, insbesondere ein oberflächlich modifiziertes Kollagen, innerhalb des nativen Knochenverbundmaterials zu erhalten, welches sicher und nicht immunogen ist. Das bedeutet, dass das atelopeptidierte Knochenverbundmaterial bereits hoch biokompatibel ist, so dass dieses bereits eine verminderte Abwehrreaktion bei einem Patienten hervorruft.

Im Rahmen der Erfindung ist es als Wesentlich erkannt worden, dass die durch die Atelopeptidierung des Knochenverbundmaterials erreichte hohe Biokompatibilität desselben noch verbessert bzw. gesteigert werden kann, indem das Knochenverbundmaterial sterilisiert wird. Der Begriff "Sterilisation" betrifft ein physikalisches Verfahren zur Entfernung und/oder Abtötung von Proteinen, von Mikroorganismen, bevorzugt in allen Entwicklungsstadien, von Sporen, von Viren, von Prionen, von Plasmiden und/oder von DNA-Fragmenten in dem Knochenverbundmaterial; bevorzugt von Kollagen, noch mehr bevorzugt atelopeptidiertes Kollagen. Gemäß der Erfindung wird die Sterilisation bei einer Temperatur von 250 °C bis 500 °C durchgeführt. Weiter bevorzugt wird die Sterilisation bei einer Temperatur von 260 °C, 270 °C, 280 °C, 290 °C, 300 °C, 310 °C, 320 °C, 330 °C, 340 °C, 350 °C, 360 °C, 370 °C, 380 °C, 390 °C, 400 °C, 410 °C, 420 °C, 430 °C, 440 °C, 450 °C, 460 °C, 470 °C, 480 °C, 490 °C oder 500 °C. Bevorzugt erfolgt die Sterilisation bei mindestens einer der zuvor genannten Temperatur, das heißt bei mindestens 250 °C bis mindestens. 490 °C. Alternativ bevorzugt erfolgt die Sterilisation maximal bei einer der zuvor genannten Temperatur, das heißt bei maximal 260°C bis maximal 500°C. Noch mehr bevorzugt ist eine beliebige Kombination der zuvor genannten minimalen und der zuvor genannten maximalen Temperatur denkbar, das heißt in einem Bereich von mindestens 250 °C bis maximal 500 °C, bevorzugt von mindestens 250 °C bis maximal 270 °C.

Es ist als wesentlich erkannt worden, dass die Sterilisation bevorzugt bei Normaldruck (ca. 1 bar) und über eine bestimmte Zeit durchgeführt wird. Weiter bevorzugt wird die Sterilisation bei einem im Vergleich zum Normaldruck niedrigen Druck, beispielsweise bei 0,5 bar, 0,6 bar, 0,7 bar, 0,8 bar oder 0,9 bar, oder bei einem höheren Druck, beispielsweise bei 1,5 bar, 2,0 bar, 2,5 bar oder 3,0 bar durchgeführt. Weiterhin bevorzugt wird die Sterilisation für 0,5, 1,0, 1,5, 2,0, 2,5, 3,0, 3,5, 4,0, 4,5, 5,0, 5,5, 6,0, 6,5, 7,0, 7,5, 8,0, 8,5, 9,0, 9,5, 10,0 oder mehr Stunden durchgeführt. Noch mehr bevorzugt wird die Sterilisation für 2 bis 5 Stunden durchgeführt.

Es ist weiterhin als wesentlich erkannt worden, dass die Sterilisation bei einer Temperatur bzw. in einem Temperaturbereich durchgeführt wird, welche eine Keramisierung des Knochenverbundmaterials verhindert, so dass dadurch das erhaltene Knochenersatzmaterial eine sehr gute sterile Formbarkeit aufweist, belastungsstabil und/oder stabil und langfristig integrierbar und/oder implantierbar ist.

Im Rahmen der Erfindung ist es des Weiteren erkannt worden, dass das erfindungsgemäße Verfahren zur Herstellung des nativen Knochenersatzmaterials mindestens die zuvor genannten Schritte (a) bis (d) umfasst. Einem Fachmann ist es allerdings verständlich, dass das Verfahren weitere Schritte umfassen kann, wie beispielsweise, jedoch keinesfalls ausschließlich, einen oder weitere Schritte zum Zerkleinern, Reinigen, Waschen, Atelopeptidieren, Entfetten, Entwässern, Trocknen, Lyophilisieren, Sterilisieren und/oder Verpacken des Knochenverbundmaterials und/oder des dadurch erhaltenen Knochenersatzmaterials. Dabei ist es denkbar, dass jeder Schritt mindestens einmal oder mehrmals wiederholbar ist, wobei die konkrete Durchführung jedes einzelnen Schrittes im Rahmen des fachmännischen Könnens liegt. Weiterhin bevorzugt erfolgt die Sterilisation durch ein physikalisches, beispielsweise ein thermisches Verfahren und/oder durch Bestrahlung, und/oder durch ein chemisches Verfahren. Bevorzugt wird zwischen den Schritten (a) und (b) ein chemisches Sterilisieren des Knochenverbundmaterials aus Schritt (a) und/oder zwischen den Schritten (b) und (c) ein Lyophilisieren des Knochenverbundmaterials aus Schritt (b) durchgeführt.

Der Begriff "Biokompatibilität" beschreibt die zwingende Voraussetzung für eine gute Langzeitverträglichkeit des nativen Knochenersatzmaterials und bedeutet, dass dieses im tierischen und/oder menschlichen Organismus in höchstem Maße verträglich ist und bevorzugt keinen negativen Einfluss auf diesen ausübt. Das bedeutet, dass von dem nativen Knochenersatzmaterial keine Toxizität, keine Teratogenität, keine Mutagenität und/oder keine Kanzerogenität ausgeht, wobei dessen Immunogenität in höchstem Maße verhindert und/oder unterdrückt wird. Das heißt, dass das erhaltene Knochenersatzmaterial maximal 0,1 % immunogen, bevorzugt maximal 0,05 %, 0,01 %, 0,005 %, 0,001°%, 0,0005 %, 0,0001 % oder 0 % immunogen ist. Der Begriff "Immunogenität" beschreibt die Eigenschaft eines Stoffes, im tierischen und/oder menschlichen Organismus eine als Immunantwort bezeichnete Reaktion des Immunsystems auszulösen, wie beispielsweise eine allergische Reaktion und/oder Abstoßungsreaktion.

Der Begriff "Osteogenese" betrifft die dauerhafte Entstehung, Bildung und/oder Regeneration neuen Knochens in, an und/oder durch das Knochenersatzmaterial, wobei der Knochen bevorzugt die Funktion des Knochenersatzmaterials übernimmt.

Der Begriff "Osteokonduktion" betrifft die passive Eigenschaft des nativen Knochenersatzmaterials, dass dessen Oberfläche, Hohlraumvolumen und/oder Gerüst das Einwachsen von Gefäßen, die Einwanderung und/oder das Anhaften von Osteoblasten und/oder Bindegewebszellen und damit den An- und/oder Aufbau von Knochen, sowie die Stabilisierung des Defektes ermöglicht. Auf diese Weise wird eine schnelle, erfolgreiche und/oder verlässliche knöcherne Durchbauung des skelettalen Defektes erreicht, um insbesondere eine Durchwachsung des Defektes mit Weichgewebe, in Verbindung mit den damit bekannten Nachteilen, zu verhindern. Wesentlicher Parameter für die Osteokonduktion ist die "Porosität" des nativen Knochenersatzmaterials, welche das Verhältnis von Hohlraumvolumen zu Gesamtvolumen beschreibt. Bevorzugt ist die Porosität anforderungsgemäß, das heißt dass beispielsweise die Anzahl an Hohlraumvolumen und/oder der Durchmesser der Poren entsprechend der gewünschten und/oder benötigten Bedingungen veränderbar ist.

Der Begriff "Resorbierbarkeit" betrifft die Eigenschaft des nativen Knochenersatzmaterials, innerhalb eines gewissen Zeitraumes nicht, teilweise oder vollständig mit körpereigenem Knochen ersetzt zu werden. Bei dem teilweisen oder vollständigen Ersatz mit körpereigenem Knochen hat es sich als vorteilhaft erwiesen, dass dieser bevorzugt innerhalb eines gewissen Zeitraums erfolgt. Dies bringt den Vorteil mit sich, dass die Wartezeiten, insbesondere in der zahnärztlichen Chirurgie, Implantologie und/oder Paradontologie, drastisch verkürzt sind. Dies bietet den weiteren Vorteil, dass die Behandlung eines Patienten in einem kürzeren Zeitraum abgeschlossen werden kann. Bevorzugt erfolgen die Resorption des Knochenersatzmaterials und der Aufbau des körpereigenen Knochens hierbei mit gleicher Geschwindigkeit, so dass damit die Ausbildung von bindegewebigen, biomechanisch minderwertigen Strukturen vollständig verhindert wird. Noch mehr bevorzugt erfolgt die Resorption zellvermittelt biologisch und/oder durch chemisch-physikalische Lösungsvorgänge. Alternativ bevorzugt ist es auch denkbar, dass das native Knochenersatzmaterial nicht resorbierbar ist, das heißt dass keine Umbildung des nativen Knochenersatzmaterials in neuen Knochen erfolgt, wobei eine knöcherne Reparation durch Osseointegration des Knochenersatzmaterials unter Bildung einer innigen Verbindung desselben mit dem Knochen denkbar ist.

Mittels des erfindungsgemäßen Verfahrens, insbesondere der erfindungsgemäßen Kombination der Atelopeptidierung des in dem Knochenverbundmaterial enthalten Kollagens, bevorzugt des Typ I Kollagens, und der anschließenden thermischen Sterilisation desselben, werden die organischen Komponenten des nativen Knochenverbundmaterials vollständig entfernt, so dass vorteilhafterweise ein biokompatibles, in höchstem Maße immunogen-freies, -unterdrückendes und/oder - verhinderndes, bioaktives, natives Knochenersatzmaterial erhalten wird.

Auf diese Weise wird es gewährleistet, dass ein sicheres, peptidfreies und gleichzeitig kollagenhaltiges natives Knochenersatzmaterial erhältlich ist, so dass auf die zusätzliche und/oder präventive Verabreichung antibiotischer und/oder immunsupressiver Mittel vollständig verzichtet werden kann.

Das erhaltene native Knochenersatzmaterial ist vorteilhafterweise einfach, kostengünstig, steril und in unbegrenzten Volumina herstellbar, wobei dieses eine einfache Applikation, Bearbeitung, Verarbeitung und/oder Formbarkeit durch einen Anwender bei Gewährleistung und Sicherstellung aller anforderungsgemäßen, eingangs genannten Anforderungen gewährleistet. Gleichzeitig ist es sichergestellt, dass dieses dem körpereigenen Knochen gleiche biomechanische Eigenschaften aufweist, wobei die Formstabilität insbesondere für größere Defekte wesentlich ist.

Vorteilhafte Weiterbildungen der Erfindung, welche einzeln oder in Kombination realisierbar sind, sind in den Unteransprüchen dargestellt.

In einer Weiterbildung der Erfindung ist es denkbar, dass das Verfahren weiterhin umfasst:
(e) Beschichten des Knochenersatzmaterials aus Schritt (d) mit einem nativen, zellbindenden Atelokollagen oder mit einem Gemisch umfassend natives, zellbindendes Atelokollagen und Hyaluronsäure; und
(f) Anlagern mindestens eines Hilfsstoffs an das Knochenersatzmaterial aus Schritt (e), wobei der Hilfsstoff ausgewählt ist aus der Gruppe umfassend mitogene, angiogene und/oder morphogene Faktoren, bakteriostatische, antibiotische und/oder pharmakologische Wirkstoffe und/oder Stammzellen.

Im Rahmen der Erfindung hat es sich als Vorteil herausgestellt, dass das Knochenersatzmaterial mit einem nativen, zellbindenden Atelokollagen beschichtet wird, welches vorteilhafterweise absolut, das heißt zu 100 % biokompatibel ist. Dies liegt darin begründet, dass es bevorzugt in flüssiger Lösung hergestellt wurde und dadurch die Atelopeptidierung nicht nur oberflächlich, sondern systemisch erfolgte. Weiterhin bevorzugt ist das Knochenersatzmaterial mit einem Gemisch umfassend natives, zellbindendes Atelokollagen und Hyaluronsäure beschichtbar. Hyaluronsäure ist einem Fachmann bekannt und ist, wie bereits im Stand der Technik offenbart, für die Neovaskularisation wichtig. Zudem weist es, ebenso wie Hydroxylapatit und alelopeptidiertes Kollagen bzw. Atelokollagen, eine natürliche antibakterielle Wirkung auf, welche bei Kombination dieser vorteilhafterweise drastisch verstärkt wird. Bevorzugt beträgt der Anteil an Hyaluronsäure in dem Gemisch 1 Vol.-%, 2 Vol.-%, 3 Vol.-%, 4 Vol.-%, 5 Vol.-%, 6 Vol.-%, 7 Vol.-%, 8 Vol.-%, 9 Vol.-%, 10 Vol.-% oder mehr Vol.-%.

Im Rahmen der Erfindung sind "mitogene Faktoren" die Zellteilung anregende Proteine, wie beispielsweise, jedoch keinesfalls ausschließlich, Wachstumsfaktoren, wie PDGF (Platelet-derived growth factor), TGF-β1 (Transforming growth factor), TGF-β2, EGF (Epidermal growth factor, IGF (Insulin-like growth factor).

Im Rahmen der Erfindung sind "angiogene Faktoren" das Wachstum von Gefäßen und/oder die Ausbildung von Gefäßstrukturen fördernde Proteine, wie beispielsweise, jedoch keinesfalls ausschließlich, Wachstumsfaktoren, wie FGF (Fibroblast growth factor) und/oder VEGF (Vascular endothelial growth factor).

Im Rahmen der Erfindung sind "morphogene Faktoren" Muster- und/oder Struktur-bildende Proteine, beispielsweise, jedoch keinesfalls ausschließlich, solche mit osteoinduktiver Wirkung, wie BMP 2 (Bone morphogenetic protein 2), BMP 3, BMP 4, BMP 5, BMP 6, BMP 7 und/oder BMP 9.

Im Rahmen der Erfindung sind "Stammzellen" Zellen, welche die Fähigkeit zur Ausdifferenzierung in verschiedene Zelltypen und/oder Gewebe aufweisen. Bevorzugt sind die Stammzellen embryonale oder adulte Stammzellen, wie beispielsweise tierische und/oder pflanzliche Stammzellen. Alternativ bevorzugt sind die Stammzellen adulte, humane Stammzellen.

Mittels dieser Ausgestaltung des Verfahrens ist es möglich, ein Osteogenese förderndes, das heißt osteoinduktives, Knochenersatzmaterial zu erhalten, welches zusätzlich ein Koagulum stabilisiert, einen Defekt auffüllt und/oder zu dessen mechanischer Festigkeit beiträgt. Zudem wird es auf dieser Weise vorteilhafterweise erreicht, dass die Knochenneubildung mit einem direkten Knochenverbund einhergeht.

Der Begriff "Osteoinduktion" betrifft die Eigenschaft des nativen Knochenersatzmaterials, die Osteogenese zu stimulieren, zu bewirken und/oder zu steigern. Dieser Effekt beruht bevorzugt auf einer Freisetzung lokal und/oder global wirksamer Faktoren, wie beispielsweise Wachstums- und/oder Reifungsfaktoren, welche in und/oder an dem nativen Knochenersatzmaterial vorhanden sind. So ist es zudem denkbar, dass das native Knochenersatzmaterial körpereigene Thrombozyten anzieht und/oder die Ansiedelung dieser auf dem Knochenersatzmaterial unterstützt. Diese Thrombozyten werden bevorzugt degradiert und enthalten, noch mehr bevorzugt mitogene, angiogene und/oder morphogene Faktoren, welche eine regenerative Aktivität aufweisen.

In einer alternativen Ausgestaltung ist es denkbar, dass das Knochenverbundmaterial bovines, porcines, equines, ovines, caprines, murines, leporines, canines und/oder felines Gewebe ist. Diese Ausgestaltung bietet den Vorteil, dass das Knochenverbundmaterial kostengünstig und in großen Mengen erhältlich ist.

In noch einer alternativen Ausgestaltung ist es denkbar, dass das Knochenverbundmaterial ausgewählt ist aus der Gruppe umfassend Knochen, insbesondere Kortikalis und/oder Spongiosa, Sehne, insbesondere Achillessehne, Band, insbesondere Kreuzband, Knorpel, insbesondere hyaliner Knorpel, elastischer Knorpel und/oder Faserknorpel, und/oder eine Mischung daraus. Diese Ausgestaltung bietet den Vorteil, dass das Knochenverbundmaterial kostengünstig und in großen Mengen erhältlich ist.

In noch einer Weiterbildung ist es denkbar, dass das native Knochenersatzmaterial mindestens 50 Gew.-% Hydroxylapatit und mindestens 20 Gew.-% atelopeptidiertes Kollagen bzw. Atelokollagen enthält. Bevorzugt weist das native Knochenersatzmaterial mindestens
51 Gew.-%, 52 Gew.-%, 53 Gew.-%, 54 Gew.-%, 55 Gew.-%,
56 Gew.-%, 57 Gew.-%, 58 Gew.-%, 59 Gew.-%, 60 Gew.-%,
61 Gew.-%, 62 Gew.-%, 63 Gew.-%, 64 Gew.-%, 65 Gew.-%,
66 Gew.-%, 67 Gew.-%, 68 Gew.-%, 69 Gew.-%, 70 Gew.-%,
71 Gew.-%, 72 Gew.-%, 73 Gew.-%, 74 Gew.-%, 75 Gew.-%,
76 Gew.-%, 77 Gew.-%, 78 Gew.-%, 79 Gew.-% oder 80 Gew.-% Hydroxylapatit auf. Bevorzugt weist das native Knochenersatzmaterial mindestens 21 Gew.-%, 22 Gew.-%, 23 Gew.-%, 24 Gew.-%, 25 Gew.-%,
26 Gew.-%, 27 Gew.-%, 28 Gew.-%, 29 Gew.-%, 30 Gew.-%,
31 Gew.-%, 32 Gew.-%, 33 Gew.-%, 34 Gew.-%, 35 Gew.-%,
36 Gew.-%, 37 Gew.-%, 38 Gew.-%, 39 Gew.-%, 40 Gew.-%,
41 Gew.-%, 42 Gew.-%, 43 Gew.-%, 44 Gew.-%, 45 Gew.-%,
46 Gew.-%, 47 Gew.-%, 48 Gew.-%, 49 Gew.-% oder 50 Gew.-% Kollagen auf. Noch mehr bevorzugt ergänzen sich die Gew.-%-Angaben von Hydroxylapatit und atelopeptidiertem Kollagen bzw. Atelokollagen zu mindestens 90 Gew.-%, 91 Gew.-%, 92 Gew.-%, 93 Gew.-%, 94 Gew.-%,
95 Gew.-%, 96 Gew.-%, 97 Gew.-%, 98 Gew.-%, 99 Gew.-%,
99,5 Gew.-%, 99,9 Gew.-% oder 100 Gew.-%.

In noch einer weiteren Weiterbildung ist es denkbar, dass das native Knochenersatzmaterial flüssig, bevorzugt als Gel, oder fest, bevorzugt als Pulver, als Granulat oder als Block, ist. Mittels dieser Ausgestaltung ist es denkbar, dass das erhaltene Knochenersatzmaterial optimal an die anforderungsgemäßen Bedingungen, beispielsweise die entsprechende prophylaktische und/oder therapeutische Behandlung, anpassbar ist.

Es wird davon ausgegangen, dass die Definitionen und/oder die Ausführungen der oben genannten Begriffe für alle in dieser Beschreibung im Folgenden beschriebenen Aspekte gelten, sofern nichts anderes angegeben ist.

Erfindungsgemäß ist weiterhin ein natives Knochenersatzmaterial zur Förderung der Osteogenese, umfassend natives Knochenverbundmaterial mit einer Hydroxylapatit-Struktur und Kollagen, wobei das Knochenersatzmaterial mittels Atelopeptidierung des Knochenverbundmaterials, insbesondere des Kollagens, und anschließender thermischer Sterilisation desselben erhältlich ist, wobei das Knochenersatzmaterial maximal 0,1 % immunogen ist.

Das erfindungsgemäße native Knochenersatzmaterial beruht auf dem Grundgedanken, dass die Kombination der Atelopeptidierung des Knochenverbundmaterials und der anschließenden thermischen Sterilisation desselben dazu führt, dass das dadurch erhaltene Knochenersatzmaterial maximal 0,1 % immunogen und damit in höchstem Maße biokompatibel ist. Bevorzugt ist das erhältliche Knochenersatzmaterial maximal 0,05 %, 0,01 %, 0,005 %, 0,001°%, 0,0005 %, 0,0001 % oder 0 % immunogen. Das bedeutet, dass das native Knochenersatzmaterial bevorzugt frei von der immunogenen terminalen Peptidkette innerhalb des Kollagenmoleküls ist, wodurch eine im Vergleich zu bisher aus dem Stand der Technik erhältlichen nativen Knochenersatzmaterialien deutlich verbesserte bzw. höchste Biokompatibilität erreichbar ist. Vorteilhafterweise kann dabei auf die zusätzliche und/oder präventive Verabreichung antibiotischer und/oder immunsupressiver Mittel vollständig verzichtet werden, da das native Knochenersatzmaterial der vorliegenden Erfindung keine allergische Reaktion und/oder Abstoßungsreaktion bei einem Patienten hervorruft.

Der Begriff "Förderung der Osteogenese" betrifft die schnelle, zuverlässige und/oder dauerhafte Entstehung, Bildung und/oder Regeneration neuen Knochens in, an und/oder durch das Knochenersatzmaterial, wobei der Knochen bevorzugt die Funktion des Knochenersatzmaterials übernimmt. Vorteilhafterweise wird dadurch eine signifikante zeitliche Verkürzung der Dauer der Osteogenese erreicht, so dass, insbesondere durch die geförderte Knochenheilung und/oder Knochenneubildung, die Wartezeit deutlich verkürzt ist. Im Rahmen der Erfindung ist es dabei erkannt worden, dass mittels der Kombination einer Atelopeptidierung und anschließender thermischer Sterilisation eines nativen Knochenverbundmaterials ein natives Knochenersatzmaterial erhältlich ist, welches die eingangs genannten Eigenschaften aufweist.

Vorteilhafte Weiterbildungen der Erfindung, welche einzeln oder in Kombination realisierbar sind, sind in den Unteransprüchen dargestellt.

In einer Weiterbildung der Erfindung ist es denkbar, dass das Knochenersatzmaterial mit einem nativen, zellbindenden Atelokollagen oder mit einem Gemisch umfassend natives, zellbindendes Atelokollagen und Hyaluronsäure beschichtbar und zur Anlagerung mindestens eines Hilfsstoffs geeignet ist, wobei der Hilfsstoff ausgewählt ist aus der Gruppe umfassend mitogene, angiogene und/oder morphogene Faktoren, bakteriostatische, antibiotische und/oder pharmakologische Wirkstoffe und/oder Stammzellen.

Im Rahmen der Erfindung hat es sich als Vorteil herausgestellt, dass das Knochenersatzmaterial mit einem 100 % biokompatiblen, nativen, zellbindenden Atelokollagen oder mit einem Gemisch umfassend 100 % biokompatibles, natives, zellbindendes Atelokollagen und Hyaluronsäure beschichtbar ist. Dabei weist das mit Atelokollagen und Hyaluronsäure beschichtete Knochenersatzmaterial eine natürliche antibakterielle Wirkung auf. Bevorzugt beträgt der Anteil an Hyaluronsäure in dem Gemisch 1 Vol.-%, 2 Vol.-%, 3 Vol.-%, 4 Vol.-%, 5 Vol.-%, 6 Vol.-%, 7 Vol.-%, 8 Vol.-%, 9 Vol.-%, 10 Vol.-% oder mehr Vol.-%.

Mittels dieser Ausgestaltung des nativen Knochenersatzmaterials ist es möglich, ein Osteogenese förderndes, das heißt osteoinduktives, Knochenersatzmaterialien zu erhalten, welche zusätzlich das Koagulum stabilisieren, den Defekt auffüllen und/oder zu dessen mechanischer Festigkeit beitragen. Auf dieser Weise wird es vorteilhafterweise erreicht, dass die Knochenneubildung mit einem direkten Knochenverbund einhergeht.

In einer alternativen Ausgestaltung ist es denkbar, dass das Knochenverbundmaterial bovines, porcines, equines, ovines, caprines, murines, leporines, canines und/oder felines Gewebe ist. Diese Ausgestaltung bietet den Vorteil, dass das Knochenverbundmaterial kostengünstig und in großen Mengen erhältlich ist.

In noch einer alternativen Ausgestaltung ist es denkbar, dass das Knochenverbundmaterial ausgewählt ist aus der Gruppe umfassend Knochen, insbesondere Kortikalis und/oder Spongiosa, Sehne, insbesondere Achillessehne, Band, insbesondere Kreuzband, Knorpel, insbesondere hyaliner Knorpel, elastischer Knorpel und/oder Faserknorpel, und/oder eine Mischung daraus. Diese Ausgestaltung bietet den Vorteil, dass das Knochenverbundmaterial kostengünstig und in großen Mengen erhältlich ist.

In noch einer Weiterbildung ist es denkbar, dass das native Knochenersatzmaterial mindestens 50 Gew.-% Hydroxylapatit und mindestens 20 Gew.-% atelopeptidiertes Kollagen bzw. Atelokollagen enthält. Bevorzugt weist das native Knochenersatzmaterial mindestens
51 Gew.-%, 52 Gew.-%, 53 Gew.-%, 54 Gew.-%, 55 Gew.-%,
56 Gew.-%, 57 Gew.-%, 58 Gew.-%, 59 Gew.-%, 60 Gew.-%,
61 Gew.-%, 62 Gew.-%, 63 Gew.-%, 64 Gew.-%, 65 Gew.-%,
66 Gew.-%, 67 Gew.-%, 68 Gew.-%, 69 Gew.-%, 70 Gew.-%,
71 Gew.-%, 72 Gew.-%, 73 Gew.-%, 74 Gew.-%, 75 Gew.-%,
76 Gew.-%, 77 Gew.-%, 78 Gew.-%, 79 Gew.-% oder 80 Gew.-% Hydroxylapatit auf. Bevorzugt weist das native Knochenersatzmaterial mindestens 21 Gew.-%, 22 Gew.-%, 23 Gew.-%, 24 Gew.-%, 25 Gew.-%,
26 Gew.-%, 27 Gew.-%, 28 Gew.-%, 29 Gew.-%, 30 Gew.-%,
31 Gew.-%, 32 Gew.-%, 33 Gew.-%, 34 Gew.-%, 35 Gew.-%,
36 Gew.-%, 37 Gew.-%, 38 Gew.-%, 39 Gew.-%, 40 Gew.-%,
41 Gew.-%, 42 Gew.-%, 43 Gew.-%, 44 Gew.-%, 45 Gew.-%,
46 Gew.-%, 47 Gew.-%, 48 Gew.-%, 49 Gew.-% oder 50 Gew.-% Kollagen auf. Noch mehr bevorzugt ergänzen sich die Gew.-%-Angaben von Hydroxylapatit und atelopeptidiertem Kollagen bzw. Atelokollagen zu mindestens 90 Gew.-%, 91 Gew.-%, 92 Gew.-%, 93 Gew.-%, 94 Gew.-%,
95 Gew.-%, 96 Gew.-%, 97 Gew.-%, 98 Gew.-%, 99 Gew.-%,
99,5 Gew.-%, 99,9 Gew.-% oder 100 Gew.-%.

In noch einer weiteren Weiterbildung ist es denkbar, dass das native Knochenersatzmaterial flüssig, bevorzugt als Gel, oder fest, bevorzugt als Pulver, als Granulat oder als Block, ist. Mittels dieser Ausgestaltung ist es denkbar, dass das erhaltene Knochenersatzmaterial optimal an die anforderungsgemäßen Bedingungen, beispielsweise die entsprechende prophylaktische und/oder therapeutische Behandlung, anpassbar ist.

Es wird davon ausgegangen, dass die Definitionen und/oder die Ausführungen der oben genannten Begriffe für alle in dieser Beschreibung im Folgenden beschriebenen Aspekte gelten, sofern nichts anderes angegeben ist.

Erfindungsgemäß ist weiterhin natives Knochenersatzmaterial zur Verwendung bei der prophylaktischen und/oder therapeutischen Behandlung entzündlicher, unfallbedingter, operativer, tumorbedingter, dysmorphischer und/oder posttraumatischer skelettaler Defekten vorgeschlagen.

Erfindungsgemäß ist des Weiteren natives Knochenersatzmaterial zur Verwendung in der Chirurgie, der Unfallchirurgie, der Traumatologie, der Neurochirurgie, der Plastischen Chirurgie, der Mund-, Kiefer- und Gesichtschirurgie, der Orthopädie, der Zahnheilkunde, der Implantologie und/oder der Parodontologie vorgeschlagen.

Der Begriff "therapeutische Behandlung" bedeutet, dass der skelettale Defekt, auf welchen in Übereinstimmung mit der vorliegenden Erfindung Bezug genommen wird, mindestens teilweise oder bevorzugter sogar vollständig in einem statistisch signifikanten Teil von Patienten behoben wird. Ob ein Teil statistisch signifikant ist, kann ohne weiteres durch den Fachmann mit verschiedenen bekannten statistischen Auswertungswerkzeugen bestimmt werden, zum Beispiel durch Bestimmung von Konfidenzintervallen, p-Wert Bestimmung, Student's t-Test und/oder Mann-Whitney-Test. Bevorzugte Konfidenzintervalle sind mindestens 90 %, mindestens 95 %, mindestens 97 %, mindestens 98 % oder mindestens 99 %. Die p-Werte sind vorzugsweise 0,1, 0,05, 0,01, 0,005 oder 0,0001. Der Begriff "prophylaktische Behandlung", wie er hier verwendet wird, bedeutet, dass die Entstehung und/oder Verschlechterung des skelettalen Defekts in einem statistisch signifikanten Teil von Patienten verhindert und/oder verlangsamt wird.

Der Begriff "Patient", wie er hierin verwendet wird, bezieht sich vorzugsweise auf Säugetiere, noch mehr bevorzugt auf Menschen.

Der Begriff "skelettaler Defekt" betrifft ein Defizit, eine Fehlbildung und/oder eine Erkrankungen mindestens eines Knochens, sowie der umgebenden Gefäße und/oder Gewebe eines tierischen und/oder menschlichen Organismus. Der Defekt kann beispielsweise systemisch, regional, angeboren und/oder erworben sein. Bevorzugt ist der skelettale Defekt ein entzündlicher, unfallbedingter, operativer, tumorbedingter, dysmorphischer und/oder posttraumatischer Defekt, welcher beispielsweise durch genetische Prozesse, durch eine Operationen, beispielsweise eine operative Entfernung eines Knochens, sowohl teilweise als auch vollständig, einen Unfall und/oder einen Sturz, durch eine Entzündung, durch infektiöse, hypoxische, toxische, medikamentöse und/oder hormonelle Faktoren bedingt ist. Der Defekt betrifft beispielsweise, jedoch keinesfalls ausschließlich, einen intraossären Defekt in der Knochendifferenzierung, der Knochenlänge, der Körpergröße, des Volumens des Knochens und/oder der Knochenstruktur, einen Knochendefekt, einen parodontalen und/oder periimplantären Defekt, eine Knochenzyste, einen fehlenden Knochenteil, eine Elevation der Sinusbasis, eine vertikale und/oder horizontale Augmentation, eine entzündliche und/oder eine degenerative Erkrankung, wie Osteoporose, Arthrose und/oder Arthritis, sowie damit verbunden ein Koagulum, eine Verletzungen der Blutgefäße und/oder eine Blutung.

Im Rahmen der vorliegenden Erfindung ist die Behandlung des skelettalen Defekts beispielsweise, jedoch keinesfalls ausschließlich, durch Auffüllung, Stabilisierung, Regeneration und/oder Rekonstruktion des skelettalen Defekts, beispielsweise durch Implantation und/oder Integration des nativen Knochenersatzmaterials in und/oder an dem Defekt, denkbar. Im Rahmen der vorliegenden Erfindung ist die Behandlung des skelettalen Defekts zudem, beispielsweise, jedoch keinesfalls ausschließlich, durch äußere und/oder innere Verabreichung des nativen Knochenersatzmaterials, beispielsweise als Nahrungsergänzungsmittel und/oder als Nahrungsmittelzusatz, denkbar.

Mittels der erfindungsgemäßen Verwendung des nativen Knochenersatzmaterials wird es erreicht, dass der Defekt aufgrund der Beitragung zu dessen mechanischer Stabilisierung und/oder Festigkeit, der Förderung der Knochenheilung und/oder der Knochenneubildung und/oder der Regeneration von Knochen- und/oder Weichgewebe, sowie damit verbunden der Hämostase, der Stabilisierung des Koagulums und/oder der Förderung der Wundheilung, schnell, erfolgreich und/oder verlässlich behandelt wird. Dabei ist es bevorzugt, dass ein knöcherner Defekt durchbaut wird, um insbesondere eine Durchwachsung des knöchernen Defektes mit Weichgewebe, in Verbindung mit den damit bekannten Nachteilen, zu verhindern.

Weitere Einzelheiten, Merkmale und Vorteile der Erfindung ergeben sich aus der nachfolgenden Beschreibung der bevorzugten Ausführungsbeispiele in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Die folgenden Ausführungsbeispiele dienen lediglich dazu, die Erfindung zu illustrieren. Sie sollen den Gegenstand der Patentansprüche in keiner Weise beschränken.

### Beispiele:

### Beispiel 1: Herstellung nativen Knochenersatzmaterials

Ein Beispiel für das erfindungsgemäße Verfahren zur Herstellung des nativen Knochenersatzmaterials umfasst die Zerkleinerung des zentralen Teils eines Rinderknochens, welcher zuvor negativ auf Bovine spongiforme Enzephalopathie (BSE) getestet wurde, sowie die Reinigung dessen Oberfläche und der Markhöhle. Das Knochenverbundmaterial weist somit eine durchschnittliche Teilchengröße von 0,01 mm bis 8 mm auf. Anschließend folgt die Extraktion von Fettkomponenten unter Verwendung von Natriumchloridlösung, Essigsäurelösung und/oder Calciumhydroxidlösung, welche, nach einer Trocknung unter vermindertem Druck, in verschiedenen Extraktionsschritten wiederholt wird. Daran schließt sich die Wiederholung der Extraktion zellulärer, globulärer Proteine in 10 %-iger wässriger Natriumchloridlösung und die Extraktion unter Verwendung wässriger Essigsäurelösungen an.

Als nächstes folgt die Atelopeptidierung des dadurch erhaltenen Knochenverbundmaterials, hier durch Auftragen und Inkubation pharmazeutisch reinen Pepsins bei einem pH-Wert von 2,5 bis 3,0 und bei 38 °C. Anschließend erfolgt eine Reinigung des atelopeptidierten Knochenverbundmaterials mit deionisiertem Wasser, um den konstanten Wasserleitfähigkeitswert einzustellen, sowie Zentrifugation der Suspension und Gefriertrocknung des Produktes bei niedrigem Druck.

Im nächsten Schritt folgt die Sterilisation des erhaltenen Knochenverbundmaterials bei Normaldruck und bei einer Temperatur von 250 °C bis 270 °C für zwei bis fünf Stunden durchgeführt wurde. Diese Sterilisation wird bei einer Temperatur von 400 °C für eine Stunde wiederholt. Das dadurch erhaltene Knochenersatzmaterial wird steril verpackt, hermetisch versiegelt und erneut durch Gammastrahlung in einer Dosis von 25 kgy sterilisiert.

### Beispiel 2: Beschichtung und Anlagerung eines Hilfsstoffs an das native Knochenersatzmaterial

Das Knochenersatzmaterial aus Beispiel wird nach seiner Sterilisation unter aseptischen Bedingungen, mit einem nativen, zellbindenden Atelokollagen beschichtet. Das Atelokollagen zeichnet sich dadurch aus, dass es absolut biokompatibel ist, da es in flüssiger Lösung hergestellt wurde und dadurch die Atelopeptidierung nicht nur oberflächlich, sondern systemisch erfolgte.

An die Atelokollagen-Beschichtung des Knochenersatzmaterials wird der Wachstumsfaktor IGF-1 angelagert, bevorzugt in einer Menge von 2,5 µg pro 1 g des Knochenersatzmaterials und sekundär durch Natriumphosphat in einer Meng von 25 µg pro 1 g des Knochenersatzmaterials unter ständiger Kontrolle des pH-Wertes. Das resultierende native Knochenersatzmaterial wird durch Rühren homogenisiert, abgefüllt, in Behältern hermetisch verschlossen und erneut sterilisiert.

## Patentansprüche

1. Verfahren zur Herstellung eines nativen Knochenersatzmaterials, umfassend:
(a) Zerkleinern und Reinigen eines nativen Knochenverbundmaterials, welches eine Hydroxylapatit-Struktur aufweist und Kollagen enthält; und
(b) Auftragen eines proteolytischen Enzyms auf das Knochenverbundmaterial aus Schritt (a), wobei das Kollagen atelopeptidiert wird; und
(c) Sterilisieren des Knochenverbundmaterials aus Schritt (b), wobei die Sterilisation bei einer Temperatur von 250 °C bis 500 °C durchgeführt wird; und
(d) Erhalten des Knochenersatzmaterials.

2. Verfahren nach Anspruch 1, weiterhin umfassend:
(e) Beschichten des Knochenersatzmaterials aus Schritt (d) mit einem nativen, zellbindenden Atelokollagen oder mit einem Gemisch umfassend natives, zellbindendes Atelokollagen und Hyaluronsäure; und
(f) Anlagern mindestens eines Hilfsstoffs an das Knochenersatzmaterial aus Schritt (e), wobei der Hilfsstoff ausgewählt ist aus der Gruppe umfassend mitogene, angiogene und/oder morphogene Faktoren, bakteriostatische, antibiotische und/oder pharmakologische Wirkstoffe und/oder Stammzellen.

3. Verfahren nach Anspruch 1 oder 2, wobei das Knochenverbundmaterial bovines, porcines, equines, ovines, caprines, murines, leporines, canines und/oder felines Gewebe ist.

4. Verfahren nach einem der Ansprüche 1 bis 3, wobei das Knochenverbundmaterial ausgewählt ist aus der Gruppe umfassend Knochen, Sehne, Band, Knorpel und/oder eine Mischung daraus.

5. Verfahren nach einem der Ansprüche 1 bis 4, wobei das native Knochenersatzmaterial mindestens 50 Gew.-% Hydroxylapatit und mindestens 20 Gew.-% atelopeptidiertes Kollagen enthält.

6. Verfahren nach einem der Ansprüche 1 bis 5, wobei das native Knochenersatzmaterial flüssig oder fest ist.

7. Natives Knochenersatzmaterial zur Förderung der Osteogenese, umfassend natives Knochenverbundmaterial mit einer Hydroxylapatit-Struktur und Kollagen, wobei das Knochenersatzmaterial mittels Atelopeptidierung des Knochenverbundmaterials, insbesondere des Kollagens, und anschließender thermischer Sterilisation desselben erhältlich ist,
**dadurch gekennzeichnet,**
**dass** das Knochenersatzmaterial maximal 0,1 % immunogen ist und
**dass** die thermische Sterilisation bei einer Temperatur von 250 °C bis 500 °C erfolgt.

8. Natives Knochenersatzmaterial nach Anspruch 7, wobei das Knochenersatzmaterial mit einem nativen, zellbindenden Atelokollagen oder mit einem Gemisch umfassend natives, zellbindendes Atelokollagen und Hyaluronsäure beschichtbar und zur Anlagerung mindestens eines Hilfsstoffs geeignet ist, wobei der Hilfsstoff ausgewählt ist aus der Gruppe umfassend mitogene, angiogene und/oder morphogene Faktoren, bakteriostatische, antibiotische und/oder pharmakologische Wirkstoffe und/oder Stammzellen.

9. Natives Knochenersatzmaterial nach Anspruch 7 oder 8, wobei das Knochenverbundmaterial bovines, porcines, equines, ovines, caprines, murines, leporines, canines und/oder felines Gewebe ist.

10. Natives Knochenersatzmaterial nach einem der Ansprüche 7 bis 9, wobei das Knochenverbundmaterial ausgewählt ist aus der Gruppe umfassend Knochen, Sehne, Band, Knorpel und/oder eine Mischung daraus.

11. Natives Knochenersatzmaterial nach einem der Ansprüche 7 bis 10, welches mindestens 50 Gew.-% Hydroxylapatit und mindestens 20 Gew.-% atelopeptidiertes Kollagen enthält.

12. Natives Knochenersatzmaterial nach einem der Ansprüche 7 bis 11, wobei das native Knochenersatzmaterial flüssig oder fest ist.

13. Natives Knochenersatzmaterial nach einem der Ansprüche 7 bis 12 zur Verwendung bei der prophylaktischen und/oder therapeutischen Behandlung entzündlicher, unfallbedingter, operativer, tumorbedingter, dysmorphischer und/oder posttraumatischer skelettaler Defekten.

14. Natives Knochenersatzmaterial nach einem der Ansprüche 7 bis 12 zur Verwendung in der Chirurgie, der Unfallchirurgie, der Traumatologie, der Neurochirurgie, der Plastischen Chirurgie, der Mund-, Kiefer- und Gesichtschirurgie, der Orthopädie, der Zahnheilkunde, der Implantologie und/oder der Parodontologie.

## Claims

1. A method for producing a native bone substitution material, the method comprising:
(a) crushing and sanitizing a native bone composite material, which has a hydroxyapatite structure and contains collagen; and
(b) applying a proteolytic enzyme on the bone composite material from step a), the collagen being atelopeptidized; and
(c) sterilizing the bone composite material from step b), the sterilization occurring at a temperature ranging from 250 °C to 500 °C; and
(d) obtaining the bone substitution material.

2. The method according to claim 1, the method further comprising:
(e) coating the bone substitution material from step d) using a native, cell-binding atelo-collagen or using a mixture comprising a native, cell-binding atelo-collagen and hyaluronic acid; and
(f) accumulating at least one auxiliary material on the bone substitution material from step e), the auxiliary material being chosen among the group of mitogenic, angiogenic and/or morphogenic factors, bacteriostatic, antibiotic and/or pharmacologic agents and/or stem cells.

3. The method according to claim 1 or 2, wherein the bone composite material is bovine, porcine, equine, ovine, caprine, murine, leporine, canine and/or feline tissue.

4. The method according to any one of the claims 1 to 3, wherein the bone composite material is chosen among the group of bone, sinew, ligament, cartilage and/or a mixture thereof.

5. The method according to any one of the claims 1 to 4, wherein the native bone substitution material contains at least 50 % by weight hydroxyapatite and at least 20 % by weight atelopeptidized collagen.

6. The method according to any one of the claims 1 to 5, wherein the native bone substitution material is liquid or solid.

7. A native bone substitution material for promoting osteogenesis, the native bone substitution material comprising a native bone composite material having a hydroxyapatite structure and collagen, wherein the bone substitution material is obtainable by atelopeptidizing the bone composite material, in particular the collagen, and by subsequently thermally sterilizing the same, **characterized in that**,
the bone substitution material is maximally 0.1 % immunogenic and **in that** the thermal sterilization occurs at a temperature of 250 °C to 500 °C.

8. The native bone substitution material according to claim 7, wherein the bone substitution material is coated with a native, cell-binding atelo-collagen or with a mixture comprising native, cell-binding atelo-collagen and hyaluronic acid and is suitable for accumulating at least one auxiliary material, the auxiliary material being chosen among the group of mitogenic, angiogenic and/or morphogenic factors, bacteriostatic, antibiotic and/or pharmacologic agents and/or stem cells.

9. The native bone substitution material according to any one of the claims 7 or 8, wherein the bone composite material is bovine, porcine, equine, ovine, caprine, murine, leporine, canine and/or feline tissue.

10. The native bone substitution material according to any one of the claims 7 to 9, wherein the bone composite material is chosen among the group of bone, sinew, ligament, cartilage and/or a mixture thereof.

11. The native bone substitution material according to any one of the claims 7 to 10, which contains at least 50 % by weight hydroxyapatite and at least 20 % by weight atelopeptidized collagen.

12. The native bone substitution material according to any one of the claims 7 to 11, wherein the native bone substitution material is liquid or solid.

13. The native bone substitution material according to any one of the claims 7 to 12 for use in the prophylactic and/or therapeutic treatment of inflammatory, accident-related, operational, tumor-related, dysmorphic and/or post-traumatic skeletal defects.

14. The native bone substitution material according to any one of the claims 7 to 12 for use in surgery, accident surgery, traumatology, neurosurgery, plastic surgery, oral and maxillofacial surgery, orthopedics, odontology, implantology and/or periodontology.

## Revendications

1. Procédé pour la production d'un matériau natif de substitution osseuse, le procédé comprenant :
(a) broyer et assainir un matériau natif de composite osseux, qui a une structure d'hydroxyapatite et contient du collagène ; et
(b) appliquer une enzyme protéolytique sur le matériau de composite osseux de l'étape a), le collagène étant atélopeptidisé ; et
(c) stériliser le matériau de composite osseux de l'étape b), la stérilisation se passant à une température allant de 250 °C à 500 °C ; et
(d) obtenir le matériau de substitution osseuse.

2. Procédé selon la revendication 1, le procédé comprenant en outre :
(e) enrober le matériau de substitution osseuse de l'étape d) d'un atélocollagène natif liant des cellules ou d'un mélange comprenant un atélocollagène natif liant des cellules et un acide hyaluronique ; et
(f) s'accumuler au moins un matériau auxiliaire sur le matériau de substitution osseuse de l'étape e), le matériau auxiliaire étant choisi parmi le groupe comprenant des facteurs mitogèniques, angiogèniques et/ou morphogèniques, des agents bactériostatiques, antibiotiques et/ou pharmacologiques et/ou des cellules souches.

3. Procédé selon la revendication1 ou 2, dans lequel le matériau de composite osseux est tissu bovin, porcin, équin, ovin, caprin, murin, lagomorphe, canin et/ou félin.

4. Procédé selon l'une quelconque des revendications 1 à 3, dans lequel le matériau de composite osseux est choisi parmi le groupe comprenant l'os, le tendon, le ligament, le cartilage et/ou un mélange de ceux-ci.

5. Procédé selon l'une quelconque des revendications 1 à 4, dans lequel le matériau natif de substitution osseuse contient au moins 50 % en poids d'hydroxyapatite et au moins 20 % en poids de collagène atélopeptidisé.

6. Procédé selon l'une quelconque des revendications 1 à 5, dans lequel le matériau natif de substitution osseuse est liquide ou solide.

7. Matériau natif de substitution osseuse pour la favorisation de l'ostéogénèse, le matériau natif de substitution osseuse comprenant un matériau natif de composite osseux ayant une structure d'hydroxyapatite et du collagène, dans lequel le matériau de substitution osseuse pouvant être obtenu par l'atélopeptidisation du matériau de composite osseux, notamment du collagène, et par la stérilisation thermique subséquente du matériau de composite osseux,
**caractériséen** ce que,
le matériau de substitution osseuse est maximalement 0,1 % immunogène et en ce que la stérilisation thermique se passe à une température allant de 250 °C à 500 °C.

8. Matériau natif de substitution osseuse selon la revendication 7, dans lequel le matériau de substitution osseuse est enrobé d'un atélocollagène natif liant des cellules ou d'un mélange comprenant un atélocollagène natif liant des cellules et un acide hyaluronique et est convient à s'accumuler au moins un matériau auxiliaire, le matériau auxiliaire étant choisi parmi le groupe comprenant des facteurs mitogèniques, angiogèniques et/ou morphogèniques, des agents bactériostatiques, antibiotiques et/ou pharmacologiques et/ou des cellules souches.

9. Matériau natif de substitution osseuse selon l'une quelconque des revendications 7 ou 8, dans lequel le matériau de composite osseux est tissu bovin, porcin, équin, ovin, caprin, murin, lagomorphe, canin et/ou félin.

10. Matériau natif de substitution osseuse selon l'une quelconque des revendications 7 à 9, dans lequel le matériau de composite osseux est choisi parmi le groupe comprenant l'os, le tendon, le ligament, le cartilage et/ou un mélange de ceux-ci.

11. Matériau natif de substitution osseuse selon l'une quelconque des revendications 7 à 10, le matériau natif de substitution osseuse contenant au moins 50 % en poids d'hydroxyapatite et au moins 20 % en poids de collagène atélopeptidisé.

12. Matériau natif de substitution osseuse selon l'une quelconque des revendications 7 à 11, dans lequel le matériau natif de substitution osseuse est liquide ou solide.

13. Matériau natif de substitution osseuse selon l'une quelconque des revendications 7 à 12 pour être utilisé dans un traitement prophylactique et/ou thérapeutique d'une anomalie squelettique inflammable, associée à un accident, opérative, associée à un tumeur, dysmorphique et/ou post-traumatique.

14. Matériau de substitution osseuse selon l'une quelconque des revendications 7 à 12 pour être utilisé en chirurgie, en chirurgie d'urgence, en traumatologie, en neurochirurgie, en chirurgie plastique, en chirurgie maxillo-faciale, en orthopédie, en odontologie, en implantologie et/ou en parodontologie.
